# EUROPEAN PATENT SPECIFICATION

(11) **EP 2 976 677 B1**
(45) Date of publication and mention of the grant of the patent: **22.04.2020**
(21) Application number: 14711523.2
(22) Date of filing: 21.03.2014
(51) Int. Cl.: A61B 6/00, H04W 4/80

(54) **METHOD FOR WIRELESS COMMUNICATION OF A DIRECT RADIOGRAPHIC PANEL**
VERFAHREN ZUR DRAHTLOSEN KOMMUNIKATION EINER DIREKT-RÖNTGEN PLATTE
PROCÉDÉ POUR LA COMMUNICATION SANS FIL D'UN PANNEAU RADIOGRAPHIQUE DIRECT

(30) Priority: 21.03.2013 BE 201300191
(43) Date of publication of application: 27.01.2016
(73) Proprietor: AGFA NV, 2640 Mortsel (BE)
(72) Inventor: EXELMANS, Walter, B-2640 Mortsel (BE)
(74) Representative: Verbrugghe, Anne Marie L.
(86) International application number: PCT/EP2014/055714
(87) International publication number: WO 2014/147225

(56) References cited:
- EP-A1- 2 322 086
- JP-A- 2005 013 310
- JP-A- 2012 034 936
- US-A1- 2006 280 337
- US-A1- 2009 194 695
- US-A1- 2010 169 423

## Description

### Field of the invention :

The present invention relates to a method or procedure for storing and sending radiographic images to a workstation. The present invention further relates to a radiographic system and a direct digital radiographic panel for application of such method.

### Background of the invention

It is known that X-rays have important applications in the field of medical imaging in which the medical-diagnostic benefit for the patient mostly largely outweighs the small and limited risk of radiation damage.

Originally, mostly silver halide-based radiographic film was used as registration medium for radiographic recording.

However, during the last decades, so-called computed radiography (CR) has increasingly gained interest. This technique uses a radiographic plate in which silver halide has been replaced as light-sensitive element by so-called storage phosphors. This method is extensively described in, for example, the Handbook of Medical Imaging, (ed. R.V. Matter et al., SPIE Press, Bellingham, 2000) corresponding to XP055101545.

Since a couple of years, direct digital radiographic techniques, known as DR (Direct Radiography), are increasingly used for radiographic recording.

This method is increasingly used as an alternative to film-based imaging techniques as well as to the above-mentioned panels based on stimulable phosphor or storage phosphor technologies.

In this direct digital radiography technique, the radiographic exposure energy is fixed pixel-wise in a radiographic sensitive panel and thereupon converted into electronic image data by means of electronic components. Subsequently, the information is read out image-wise and displayed on an appropriate monitor in order to allow a radiologist to make a diagnosis.

One of the driving forces behind the success of direct digital radiography is the ability to rapidly visualize the obtained radiographic images and to communicate them in a simple and efficient manner by means of data networks to one or more locations in order to be remotely analyzed and diagnosed by a radiologist or other medical expert. Thanks to this technique, the delays that typically occur in developing, packing and physically sending radiographic films, as well as the inconvenience related to scanning developed films and the corresponding loss of resolution, are avoided.

Direct radiographic (DR) systems have the advantage, compared to computer radiography (CR) systems based on storage or stimulable phosphors, that the latent stored radiographic image does not have to be read out (in a digitizer). On the contrary, the digital radiographic image can immediately or directly be read out in order to make a radiographic diagnosis. This diagnosis can then be carried out on a local workstation as well as on a very remotely located workstation.

Originally, the first direct radiographic panels were integrated into the complete radiographic imaging system. The wiring and cabling was then provided in a manner which minimizes the disturbance for the radiographer when putting in place the direct radiographic panel for recording a body part of the patient.

More recently, portable direct radiographic panels have been introduced into the market. These panels use built-in batteries and wireless communication with the radiographic control panel or workstation, as well as with the data storage device and display components.

Thanks to the last-mentioned aspects, such portable wireless panels can be used in a very flexible way and are very appropriate for use in a fully digital radiographic recording system.

They can be used in a hospital or a medical diagnostics centre as well as in a completely new installed radiographic imaging system, or in a so-called retrofit situation. The term "retrofit" is to be understood here as an existing radiographic imaging system which previously used radiographic films or stimulable or storage phosphor imaging plates and in which the latter means are replaced by a direct radiographic recording medium, a so-called direct radiographic or DR panel, without, for example, the workstation or the radiography source itself having to be replaced. The advantage of a retrofit radiography system, compared to a completely new installed direct radiography system, lies in its lower investment cost since part of the existing radiographic station can be kept.

Although portability and wireless communication of the radiographic recording medium are an obvious advantage when using portable and wireless DR panels, these features, however, also cause potential problems in practical conditions of use.

One of the problems encountered when using such panels, is the fact that, once the recording is finished, the stored radiographic image cannot, or only difficultly, be sent to the radiographic console due to transmission difficulties with the available wireless network.

As long as the complete radiographic image has not been transmitted to the corresponding radiographic console, the panel is not available for a subsequent recording, which results in an unnecessary prolongation of the so-called 'cycle time'. The term 'cycle time' is to be understood as the time needed to finish a complete radiographic recording, including the correct reception of the radiographic image by the console.

As a further disadvantage of such inadequate communication with the radiographic workstation, the battery of the direct radiographic panel is drained over a longer period of time during such inadequate wireless communication.

As a result of this, the battery is depleted more quickly, which in turn requires the direct radiographic panel to be charged more often in a station provided for that purpose (the so-called cradle). During this charging cycle, the direct radiographic panel is not available for radiographic recording.

### Prior art

The problems of a direct radiographic panel that wishes to transmit its radiographic image information wirelessly to an external element, but which faces transmission problems, is described, for example, in US 8,053,727, published November 8, 2011, assigned to Fujifilm Corp., Japan.

This patent also mentions the impact of such transmission problems on the ability of the on-board battery of the direct radiographic panel, as this on-board battery is hereby heavily and unnecessarily drained.

See, for example, column 2, lines 11-17, in which as an object of the invention a direct radiographic device is provided in which the battery capacity is not consumed needlessly and in which the image information can be effectively transmitted to an external element.

Column 9, first five lines, states that this object can be achieved by suspending the transmission of the image data in case such problems occur during the wireless transmission of the radiographic images.

Column 2, last lines, further describes the problems caused by the occasional inadequateness of a wireless communication between the direct radiographic panel and an external element. In case a non-optimal communication is detected, the transmission process is halted and resumed once the communication is correctly reestablished.

However, in the description of this invention, the term "external element" relates to a battery cradle, not the radiographic console itself.

According to the embodiment and invention described herein, a transceiver is used to transmit the image information from the direct radiographic panel to a corresponding transceiver of the battery cradle, stored locally therein and then forwarded to a radiographic console only after it has been stored in this battery cradle.

The communication between the battery cradle and the radiographic console preferably proceeds by a wired connection. This is also clearly evident, for example, from claim 3 which states that the battery cradle uses a wired connection to transmit the image information to the external apparatus, i.e. the radiographic console (claim 3, second part : the charging cradle transmits the image information by wire communications to the external apparatus). In this embodiment, however, even if the direct radiographic panel is placed in the charging cradle in order to charge its battery and the image is transmitted wirelessly from the DR plate to the battery cradle, the transmission will continue, even in case of a bad wireless connection, as there will be no problems with respect to a depletion of the battery of the direct radiographic panel since the battery is meanwhile being charged.

However, this method has the disadvantage that charging of the battery takes longer since the battery is additionally drained during this inadequate wireless communication.

Another disadvantage of the method described in this patent is that the radiographic image information stored in the direct radiographic panel is wirelessly transmitted from this panel to the battery cradle and is only forwarded to the console from this element.

US Patent US 8,116,599, corresponding to US 2006/0280337 A1, - published on February 20, 2012, also assigned to Fujifilm Corp., claiming priority of June 6, 2005 - also treats the problems of wirelessly transmitting radiographic image data from a direct radiographic panel to a workstation or console.

Contrary to the patent described above, this patent does not mention a battery cradle or charging cradle. In the embodiment of the invention described herein, the radiographic images are transmitted directly from the internal memory of the direct radiographic panel, through a wireless communication unit, to a console or workstation. The latter is composed of, for example, a computer and a monitor (see e.g. column 5, lines 43/44.) See also column 9, lines 9-11, in which the console can be composed of a notebook computer or similar.

However, the problems on which this invention is based are not related to a potential depletion of the battery of the direct radiographic panel.

The basis and the definition of the problems of this patent are exposed in column 2, lines 9-15 : in case of a wireless communication of data in a hospital, confidential data of a patient can be intercepted by a third person who is 'unrelated to the hospital', for example, a hacker.

The problems solved by this invention relate to securing patient data in a hospital environment by preventing that patient data, as contained in wireless communication signals, are "hijacked' by a third party ("preventing random interceptions of the wireless communication signals".)

The term "wireless communication signals" is hereinafter understood to be "signals that are wirelessly communicated".

According to the invention, this object is achieved as follows : a module is built in the direct radiographic panel which measures the distance between said panel and the module that will capture the signals, for example the console.

If this distance is below a predetermined level, the data are simply forwarded.

If this distance is above a predetermined level, the data are not forwarded, unless a prior authentication of the console takes place (for example by means of a password authentication).

The distance between the direct radiographic panel and the console is measured by measuring the time lapse of a test signal that is exchanged between the panel and the console. By means of a formula incorporating the speed of light, the distance can be calculated on the basis of the time lapse (see column 6, half way the patent).

According to the invention described in this patent, an aspect of the wireless communication to the console is controlled prior to forwarding the radiographic image data.

In a way, the distance between the direct radiographic panel and the console is related to the quality of the wireless connection between both devices.

The smaller this distance, the more likely that the quality of the connection is adequate.

If the quality is found to be adequate, the wireless communication will effectively take place, otherwise not.

The patent is silent on the problems concerning the depletion of the battery in case of a bad wireless communication.

The patent is also silent on the quality of the wireless communication as such, it only deals with the problems related to the distance between the devices and their consequences for such wireless transmission.

US Patent US 8,149,116, claiming priority of July 2008 and published on April 3, 2012, also assigned to Fujifilm Corp., also relates to a radiographic recording system in which a direct radiographic panel is used.

The above patent describes the problems of a bad connection between the direct radiographic system and the console, however not in the situation of a wireless connection, but in case of a wired connection.

The description of the problems starts with the situation that arises when the connection cable is not connected or with the situation in which the cable is connected, yet does not function adequately.

A disconnected cable can occur if the direct radiographic station is frequently respectively connected to and disconnected from this cable.

An inadequate operation can occur if the cable is, for example, incorrectly connected or if the communication with the console is inadequate for another reason.

According to the description of the prior art as discussed in this patent, hence the radiographic recording cannot start (see, for example, the passage on top of column 2).

The solution proposed for this problem consists in incorporating a determination unit and a control unit for the communication with the console, in the direct radiographic panel.

The determination unit establishes whether the connection status between the direct radiographic panel and the console (via the communication cable) is abnormal or not. In function of this result, the control unit then proceeds as follows :
- if the communication is adequate, the radiographic recording is carried out and the radiographic image is forwarded to the console;
- if the communication is inadequate, the radiographic recording is also carried out (contrary to the prior art) and the image is stored in the internal memory of the direct radiographic panel (see, for example, claim 1, column 28).

In a preferred embodiment (claim 2), a quality detection unit for the wireless communication (communication quality detection unit) is added to the direct radiographic panel, whereby said quality detection unit not only verifies the physical presence of a connection with the console, yet also controls the quality of this connection.

In further preferred embodiments, a detector or a mechanical switch is provided. Claim 6 describes that first it is checked whether a predetermined control signal is exchanged between the direct radiographic panel and the console.

However, this text is silent on the problems related to a potential negative impact on the operation or condition of the battery of the direct radiographic panel in case of an inadequate wireless communication.

On the contrary, the text describes the consequences of a bad communication between the direct radiographic panel and the console, yet the communication itself is limited to a wired connection.

The situation of wireless communication between the direct radiographic panel and the console is not discussed or described. However, the latter form of communication is of great importance from a practical point of view : in practice, the cables and wired connections cause indeed great inconvenience when using direct radiographic workstations.

It is an object of the present invention to respectively prevent and solve the above-described disadvantages and problems.

### Summary of the invention

The above-described advantages and aspects are realized by a method as described in claim 1.

Another feature of the invention relates to a direct radiographic panel as described in the independent claim 10.

Specific features of preferred embodiments of the invention are set out in the dependent claims.

Further advantages and embodiments of the present invention will become apparent from the following description.

### Description of the invention

The present invention relates to the method or procedure of claim 1 for wireless communication between a direct radiographic panel and a radiographic workstation, comprising the following steps :
- detecting, by means of a processor of the direct radiographic panel, the quality of a wireless connection between an output unit of the direct radiographic panel which can read out the radiographic image stored in the direct radiographic panel and convert it into wireless communication signals, and a reception unit of the radiographic workstation which can receive the wireless communication signals which have been sent by the output unit of the direct radiographic panel;
- if the quality of the wireless connection between the output unit of the direct radiographic panel and the reception unit of the radiographic workstation is equal to or greater than a predetermined value, converting the radiographic image stored in the direct radiographic panel into wireless communication signals and forwarding these signals to the reception unit of the radiographic workstation by means of the output unit of the direct radiographic panel;
- if the quality of the wireless connection between the output unit of the direct radiographic panel and the reception unit of the radiographic workstation is below a predetermined value, storing the radiographic image in the memory of the direct radiographic panel.

The above-described method or procedure is hereinafter in this description referred to as the 'base' method or procedure.

The present invention also relates to the direct radiographic panel of claim 10 for use in a radiographic recording. To that end, this panel comprises the following means :
- a means for detecting the quality of a wireless connection between an output unit of the direct radiographic panel which can read out the radiographic image stored in the direct radiographic panel and convert it into wireless communication signals, and a reception unit of a radiographic workstation that can receive the wireless communication signals which have been transmitted by the output unit of the direct radiographic panel;
- a means for comparing the quality of the wireless connection between the output unit of the direct radiographic panel and the reception unit of the radiographic workstation with a predetermined value;
- a means for, if the comparison reveals that the quality of the wireless connection is equal to or greater than the predetermined value, converting the radiographic image stored in the direct radiographic panel into wireless communication signals and forwarding these signals to the reception unit of the radiographic workstation by means of the output unit of the direct radiographic panel;
- a means for, if the comparison reveals that the quality of the wireless connection is below the predetermined value, storing the radiographic image in the memory of the direct radiographic panel.

### Preferred embodiments of the invention

According to a preferred embodiment of the method according to the invention, if the quality of the wireless connection between the output unit of the direct radiographic panel and the reception unit of the radiographic workstation is below a predetermined value, the radiographic images stored in the memory of the direct radiographic panel are converted into wireless communication signals and forwarded to the reception unit of the radiographic workstation by the output unit of the direct radiographic panel as soon as said quality is again equal to or greater than the predetermined value.

According to a further preferred embodiment of the invention, if the direct radiographic panel is equipped with an output unit comprising multiple, preferably three, antennas for wireless communication, the quality of the wireless connection between each of these antennas and the radiographic workstation is detected and the highest detected quality value is used for comparison with the predetermined value of the quality of the wireless connection between the direct radiographic panel and the radiographic workstation.

According to a further preferred embodiment of the invention, if the direct radiographic panel is equipped with an output unit comprising multiple antennas, the method described in the first two steps of the above-mentioned base method is repeated, and the radiographic image will only be stored in the memory of the direct radiographic panel if each quality value of the wireless connection detected in this way between each of these antennas and the radiographic workstation is below the predetermined value.

According to a further preferred embodiment of the invention, if the quality of the wireless connection between the output unit of the direct radiographic panel and the reception unit of the radiographic workstation is below a predetermined value, one or more of the following data is forwarded to the radiographic workstation : temperature of the direct radiographic panel, battery status, a low resolution radiographic image, for example one by eight pixels.

According to a further preferred embodiment of the invention, if the quality of the wireless connection between the output unit of the direct radiographic panel and the reception unit of the radiographic workstation is below a predetermined value, a check will take place to see if the radiographic image has been stored in the memory of the direct radiographic workstation, whereby a feedback of this control is provided to the user. This feedback to the user can, for example, be provided in the form of an audio or visual signal that is emitted by the direct radiographic panel, or via a signal that is transmitted from the direct radiographic panel to the radiographic workstation that in turns provides it to the user. More preferably, this feedback is only provided if the control reveals that the radiographic image is not stored in the memory of the direct radiographic panel.

According to a further preferred embodiment of the last above-described embodiment, the control status relating to the storage of the radiographic image in the memory of the direct radiographic workstation is added to the data that are forwarded to the radiographic workstation.

### Detailed description of the invention

The output unit of the direct radiographic panel reads out the radiographic image signal stored in the direct radiographic panel and converts it into wireless communication signals.

Different existing communication protocols can be used to this end, such as Bluetooth, Ultra Wide Band (UWB), HiSWANa (High Speed Wireless Access Network type a), Wireless 1394, Wireless LAN, Wireless USB etc.

The output unit of the direct radiographic panel is usually composed of an electronic chip for wireless communication that is an integral part of the direct radiographic panel.

This electronic chip in the direct radiographic panel ensures the wireless communication with the radiographic workstation as well as with the radiographic source, inter alia for forwarding the radiographic image data, and is a means that is per se known to one skilled in the art. This is described, for example, in US patents No. US 7 829 859 and the above-cited US 8 116 599, both assigned to Fuji Photo Film, Inc., Japan. The former patent describes, for example, how the direct radiographic panel, via this wireless communication module, forwards the digital image signals stored in the panel to the radiographic console by means of a transceiver provided in the panel. As an example of wireless communication, the UWB protocol (Ultra Wide Band) is mentioned. This UWB Protocol has the advantage of limiting the energy consumption and offers the possibility of applying higher communication speeds, compared to other wireless communication techniques.

The wireless communication unit can convert the image signals into wireless communication signals according to one of the following existing wireless communication protocols : UWB, Bluetooth, Zigbee, HiSWANa (High Speed Wireless Access Network type a), HiperLAN, Wireless 1394, Wireless USB, and finally Wireless LAN, infrared (irDA), NFC (Near Field Communication) and IO-Homecontrol.

Preference is given to a wireless communication protocol that operates according to the IEEE 802.11 standard.

The direct radiographic panel then communicates with the wireless network of the radiographic workstation by means of a short distance radio or infrared connection via one of the above-mentioned wireless communication protocols.

Generally, a short distance radio connection is preferred over an infrared connection since the former operates omnidirectionally, whereas in case of an infrared connection - since this is an optical connection - a direct optical path needs to be created between the sender and the receiver of the signals.

Bluetooth is to be understood as an open wireless protocol for exchanging date over short distances between fixed and mobile devices, whereby personal networks are created (PANs). Originally, Bluetooth was provided as a wireless alternative to RS-232 data cables. This wireless protocol enables to wirelessly connect different devices, without synchronization problems.

Near Field Communication is to be understood as a short distance wireless communication technology that enables the exchange of data between devices over a distance up to 4 inches. NFC combines the interface of a smart card and a reader in one single device. A NFC device can communicate with existing ISO/IEC 14443 smart cards and readers as well as with other NFC devices. It is compatible with existing wireless infrastructure and is primarily intended for use in mobile phones.

The term WI-FI is to be understood as a group of IEEE 802.11 standards for wireless communication techniques that use the same base protocol. Wi-Fi is used, inter alia, for creating wireless LANs (Local Area Networks) for computer communications.

The direct radiographic panel marketed by General Electric under the trade name Flashpad uses, for example, Ultra Wide Band. Operating with UWB instead of WI-FI has the advantage that this UWB network is a separate independent communication channel whereby the transmission of the radiographic images does not interfere with other data transmission on the WI-FI network. This promotes the speed and reliability of the data transmission of the radiographic images, yet has a couple of disadvantages.

The data transmission speed when using a UWB channel such as the one marketed by Starix is, for example, 400 Mbps.

A typical radiographic panel has a pixel image content of 3 072 x 3 072 x 2 bytes, i.e. 18 874 368 bytes, or slightly less than 20 Mbytes.

With a wired Ethernet connection with a connection speed which is typically of 100 Mbits/sec to several Gbits/sec (e.g. 6 Gbits/sec), it thus takes at most one second to forward a radiographic image of the direct radiographic panel to the radiographic workstation.

In case of a wireless connection, the time needed to forward the radiographic image from the direct radiographic panel to the radiographic workstation depends upon different factors.

The typical transmission speed of a WIFI type 802.11 b connection is 11 Mbits/sec and that of the very common 802.11g connection is 54 Mbits/sec. When using the latter standard, the time needed to forward a typical 20 Mbyte radiographic image will hence be about five seconds.

Some commercially available direct radiographic panels, such as the Canon CXDI-80C Wireless, are equipped with a newer version of the IEEE 802 standard, namely the 802.11n standard. The speed of this standard is significantly higher than the conventional 11a or 11b variants and can be up to 600 Mbits/sec. This, however, is the so-called "maximum net data rate" and in practice, the effective transmission speed will be more limited than the nominal or specified transmission speed, due to several environmental factors.

If, as a result of such environmental factors, the effective transmission speed is below a critical threshold, the effective time to transmit the radiographic image will be very long. This brings about several adverse consequences, as indicated above. First of all, the battery of the direct radiographic panel is hereby severely drained, and as a result the panel will have to be reconnected to the battery charger earlier than expected in order to be recharged.

On the other hand, the cycle time, i.e. the time needed to completely finish a radiographic recording, becomes unnecessarily long. During this total lapse of time, the respective direct radiographic panel will not be available for a next radiographic recording.

The radiographic workstation that receives the wireless communication signals that have been emitted by the output unit of the direct radiographic panel can, for example, be composed of a stationary or portable computer equipped with a monitor for displaying the radiographic recording.

This workstation is usually equipped with the necessary image processing software in order to enhance the diagnostic value of the displayed radiographic images. It can further be connected via a LAN to the PACS (Picture Archiving and Communication System) network as well as to the HIS network (Hospital Information System) of the hospital.

In a preferred embodiment of the invention, the direct radiographic panel is equipped with several antennas, more preferably three or more antennas.

In such preferred embodiment, the direct radiographic panel will detect the quality of the radiographic connection created by one of these antennas with the radiographic workstation.

If the detected value is equal to or greater than the predetermined value, the wireless communication between the radiographic workstation and the direct radiographic panel will proceed via the respective antenna.

If this is not the case, the direct radiographic panel will use one of its other antennas to create the wireless communication with the radiographic workstation.

If this wireless communication is adequate, i.e., concretely, if the value of the wireless connection thus created is above the predetermined value, the wireless communication between the radiographic workstation and the direct radiographic panel will be carried out via this last antenna.

If this is not the case, the direct radiographic panel, provided it has another unused antenna available, will use the next antenna in order to create the wireless communication with the radiographic workstation.

If this wireless communication is adequate, i.e., concretely, if the value of the wireless connection thus created is above the predetermined value, the wireless communication between the radiographic workstation and the direct radiographic panel will be carried out via this last antenna.

The method described above is repeated until one of the antennas of the direct radiographic panel has created a satisfying wireless communication with the radiographic workstation. The term "satisfying wireless communication" is to be understood as a connection whose quality is above the predetermined value.

In an alternative method, a first step consists in detecting the quality of the wireless connection between each of the antennas of the direct radiographic panel with the radiographic workstation, whereby the highest detected quality value is then used for comparison with the predetermined value of the quality of the wireless connection between the direct radiographic panel and the radiographic workstation.

Only if this highest detected value is not equal to or greater than the predetermined value of the wireless connection, the radiographic image will be stored in the memory of the direct radiographic panel.

If one or more of the detected quality values of the wireless connection between the different antennas of the direct radiographic panel and the radiographic workstation are equal to or greater than the predetermined value of the quality of the wireless connection, the wireless connection is created by the antenna with the highest detected quality value.

The radiographic image stored in the direct radiographic panel is then converted into wireless communication signals. The output unit of the direct radiographic panel then forwards these signals, via the antenna with the highest detected quality value, to the reception unit of the radiographic workstation.

A situation in which one antenna of the direct radiographic panel provides a satisfying wireless connection quality with the radiographic workstation, whereas one or more other antennas lack to do so, can occur, for example, when the direct radiographic panel is put in a radiographic bucky table. The antennas are located on different physical locations in the direct radiographic panel and it may well be possible that the metal parts of the bucky table disturb the operation of one or more antennas, yet not of another antenna of the direct radiographic panel. In this case, the latter antenna will provide, for example, a satisfying wireless connection, whereas the other antennas will not, due to the interfering presence of the metal components of the bucky table.

A direct radiographic panel equipped with different antennas is, for example, described in US 7,873,145.

In Figures 5 and 6 of this patent, a direct radiographic panel is described in which there are always three antennas linked to one transmitter.

Figure 5 shows that the three antennas are installed on different locations in the "handling section" or the lever of the DR panel.

Figure 6 shows that the three antennas are disposed at a larger distance from one another across the whole surface of the direct radiographic panel.

If the workstation is also equipped with several antennas, the communication between the direct radiographic panel and the workstation can proceed between one specific pair of antennas, i.e. one antenna of the DR panel and one antenna of the workstation. In an alternative embodiment, the communication can, however, also take place via several data channels between several pairs of antennas.

Each per se usable antenna can be used to implement the present invention. This can be a small chip antenna (with or without built-in amplifier) or an omnidirectional antenna, etc.

The quality of the wireless transmission between the output unit of the radiographic panel and the radiographic workstation can be determined by means of an evaluation of the signal-to-noise ratio of the wireless (wifi) signal in an antenna of the panel.

When the signal-to-noise ratio is below a predefined threshold, this could be the consequence of a signal weakening due to metal parts in the neighbourhood of the DR panel. For example metal parts present in a bucky device supporting the radiographic panel may be a cause of decrease of the signal strength.

Other parameters than the signal-to-noise ratio may also be used. Examples of such parameters are the number of retries to transmit a datagram which are performed. Poor wireless transmission may give rise to a corrupt transmitted signal which in its turn gives rise to a retry. The number of retries thus is an indication of the quality of the transmission.

Still other parameters may be used as an indication of transmission quality.

All such applicable parameters will be parameters the values of which either directly or indirectly depend on the signal-to-noise ratio of the transmitted signal (or may also be due to the fact that more than one transmitters use the same frequency band).

Means are commercially available to monitor this signal-to-noise ratio and occasionally to generate an indication of the determined signal-to-noise ratio.

### Exemplary embodiment :

A radiographic image of a patient is made, whereby the direct radiographic panel is put in the radiographic bucky table.

After the recording, it is assessed whether the WIFI connection between the output unit of the direct radiographic panel and the reception unit of the radiographic workstation is insufficient to be able to forward the radiographic recording within a reasonable lapse of time (the cause thereof probably lies in the presence of different metal components of the radiographic bucky table around the direct radiographic panel that can very adversely affect the quality of the wireless connection.).

Only the battery status of the direct radiographic panel, as well as a low resolution image (one by eight pixels), is wirelessly forwarded to the radiographic workstation.

The radiographic image is stored in the memory of the direct radiographic panel, which storage is confirmed to the user by means of an audio signal emitted by a buzzer. (This confirmation can also be provided via an alternative feedback signal, for example an audio signal, or another actuator that, for example, can be wirelessly activated, Zigbee, NFC, etc.)

Thanks to this signal, the radiographer or any other user knows that the direct radiographic panel is available again for a next radiographic recording, and the following patient can be treated.

The term "Zigbee" is to be understood as a specification for a series of communication protocols that use short, low energy radio waves based on the IEEE 802.15.4-2003 standard for LR-WPANs (Low Rate Wireless Personal Area Networks), such as wireless light switches for lamps and more generally end-user directed electronic devices operating via short-distance radio waves. The technology defined by the Zigbee specification is intended to be easier and less expensive to use than other WPANs, such as Bluetooth. Zigbee is intended for radio frequency applications that aim at providing a limited data transmission, a long battery life and a secured network environment.

## Claims

1. Method for wireless communication between a direct radiographic panel and a radiographic workstation, comprising the following steps :
• detecting, by means of a processor of the direct radiographic panel, the quality of a wireless connection between an output unit of the direct radiographic panel which can read out the radiographic image stored in the direct radiographic panel and convert it into wireless communication signals, and a reception unit of the radiographic workstation which can receive the wireless communication signals which have been sent by the output unit of the direct radiographic panel; wherein said quality is determined by means of a parameter the value of which depends on the signal to noise ratio of said wireless connection,
• if the quality of the wireless connection between the output unit of the direct radiographic panel and the reception unit of the radiographic workstation is equal to or greater than a predetermined value, converting the radiographic image stored in the direct radiographic panel into wireless communication signals and forwarding these signals to the reception unit of the radiographic workstation by means of the output unit of the direct radiographic panel;
• if the quality of the wireless connection between the output unit of the direct radiographic panel and the reception unit of the radiographic workstation is below a predetermined value, storing the radiographic image in the memory of the direct radiographic panel.

2. Method according to claim 1, wherein said parameter is the signal to noise ratio of said wireless transmission.

3. Method according to claim 1, wherein, if the quality of the wireless connection between the output unit of the direct radiographic panel and the reception unit of the radiographic workstation is below a predetermined value, the radiographic images stored in the memory of the direct radiographic panel are converted into wireless communication signals and forwarded to the reception unit of the radiographic workstation by the output unit of the direct radiographic panel as soon as said quality is again equal to or greater than the predetermined value.

4. Method according to claim 1, wherein, if the direct radiographic panel is equipped with an output unit comprising multiple antennas for wireless communication, the quality of the wireless connection between each of these antennas and the radiographic workstation is detected and the highest detected quality value is used for comparison with the predetermined value of the quality of the wireless connection between the direct radiographic panel and the radiographic workstation.

5. Method according to claim 1, wherein, if the direct radiographic panel is equipped with an output unit comprising multiple antennas, the method described in the first two steps of claim 1 is repeated, and the radiographic image will only be stored in the memory of the direct radiographic panel if each quality value of the wireless connection detected in this way between each of these antennas and the radiographic workstation is below the predetermined value.

6. Method according to claim 1, wherein, if the quality of the wireless connection between the output unit of the direct radiographic panel and the reception unit of the radiographic workstation is below a predetermined value, one or more of the following data is forwarded to the radiographic workstation : temperature of the direct radiographic panel, battery status, a low resolution radiographic image.

7. Method according to claim 1, wherein, if the quality of the wireless connection between the output unit of the direct radiographic panel and the reception unit of the radiographic workstation is below a predetermined value, a check will take place to see if the radiographic image has been stored in the memory of the direct radiographic workstation, whereby preferably a feedback of this control is provided to the user, whereby, more preferably, this feedback is provided in the form of an audio or visual signal that is emitted by the direct radiographic panel, or via a signal which is transmitted from the direct radiographic panel to the radiographic workstation which in turns provides it to the user.

8. Method according to claim 7, wherein the control status relating to the storage of the radiographic image in the memory of the direct radiographic workstation is added to the data that are forwarded to the radiographic workstation.

9. Method according to claim 7, wherein the feedback to the user is only provided if the control reveals that the radiographic image has not been stored in the memory of the direct radiographic panel.

10. Direct radiographic panel, comprising the following means :
• a means for detecting the quality of a wireless connection between an output unit of the direct radiographic panel which can read out the radiographic image stored in the direct radiographic panel and convert it into wireless communication signals, and a reception unit of a radiographic workstation that can receive the wireless communication signals which have been transmitted by the output unit of the direct radiographic panel;
• a means for comparing the quality of the wireless connection between the output unit of the direct radiographic panel and the reception unit of the radiographic workstation with a predetermined value;
• a means for, if the comparison reveals that the quality of the wireless connection is equal to or greater than the predetermined value, converting the radiographic image stored in the direct radiographic panel into wireless communication signals and forwarding these signals to the reception unit of the radiographic workstation by means of the output unit of the direct radiographic panel;
• a means for, if the comparison reveals that the quality of the wireless connection is below the predetermined value, storing the radiographic image in the memory of the direct radiographic panel,
• wherein said means for detecting the quality of the wireless connection is arranged to evaluate said quality by means of a parameter the value of which depends on the signal to noise ratio of said wireless transmission.

## Patentansprüche

1. Ein Verfahren zur drahtlosen Kommunikation zwischen einer Direktröntgenplatte und einer radiografischen Arbeitsstation, umfassend die folgenden Schritte:
- Detektieren, mittels eines Prozessors der Direktröntgenplatte, der Qualität einer drahtlosen Verbindung zwischen einer Ausgabeeinheit der Direktröntgenplatte, welche das in der Direktröntgenplatte gespeicherte Röntgenbild auslesen und in drahtlose Kommunikationssignale umwandeln kann, und einer Empfangseinheit der radiografischen Arbeitsstation, welche die durch die Ausgabeeinheit der Direktröntgenplatte gesendeten drahtlosen Kommunikationssignale empfangen kann, wobei die Qualität mittels eines Parameters, dessen Wert vom Signal-Rausch-Verhältnis der drahtlosen Verbindung abhängig ist, bestimmt wird,
- falls die Qualität der drahtlosen Verbindung zwischen der Ausgabeeinheit der Direktröntgenplatte und der Empfangseinheit der radiografischen Arbeitsstation größer oder gleich einem vorgegebenen Wert ist, Umwandlung des in der Direktröntgenplatte gespeicherten Röntgenbildes in drahtlose Kommunikationssignale und Übertragung dieser Signale zur Empfangseinheit der radiografischen Arbeitsstation mittels der Ausgabeeinheit der Direktröntgenplatte,
- falls die Qualität der drahtlosen Verbindung zwischen der Ausgabeeinheit der Direktröntgenplatte und der Empfangseinheit der radiografischen Arbeitsstation unter einem vorgegebenen Wert liegt, Abspeichern des Röntgenbildes im Speicher der Direktröntgenplatte.

2. Verfahren nach Anspruch 1, wobei der Parameter das Signal-Rausch-Verhältnis der drahtlosen Übertragung ist.

3. Verfahren nach Anspruch 1, wobei, falls die Qualität der drahtlosen Verbindung zwischen der Ausgabeeinheit der Direktröntgenplatte und der Empfangseinheit der radiografischen Arbeitsstation unter einem vorgegebenen Wert liegt, die im Speicher der Direktröntgenplatte abgespeicherten Röntgenbilder in drahtlose Kommunikationssignale umgewandelt und mittels der Ausgabeeinheit der Direktröntgenplatte dann zur Empfangseinheit der radiografischen Arbeitsstation übertragen werden, wenn die Qualität erneut größer oder gleich dem vorgegebenen Wert ist.

4. Verfahren nach Anspruch 1, wobei, falls die Direktröntgenplatte mit einer mehrfache Antennen für drahtlose Kommunikation umfassenden Ausgabeeinheit ausgestattet ist, die Qualität der drahtlosen Verbindung zwischen jeder dieser Antennen und der radiografischen Arbeitsstation detektiert wird und der höchste detektierte Qualitätswert zum Vergleich mit dem vorgegebenen Wert der Qualität der drahtlosen Verbindung zwischen der Direktröntgenplatte und der radiografischen Arbeitsstation benutzt wird.

5. Verfahren nach Anspruch 1, wobei, falls die Direktröntgenplatte mit einer mehrfache Antennen umfassenden Ausgabeeinheit ausgestattet ist, das in den ersten zwei Schritten von Anspruch 1 beschriebene Verfahren wiederholt wird und das Röntgenbild erst dann im Speicher der Direktröntgenplatte abgespeichert wird, wenn jeder so detektierte Qualitätswert der drahtlosen Verbindung zwischen jeder dieser Antennen und der radiografischen Arbeitsstation unter dem vorgegebenen Wert liegt.

6. Verfahren nach Anspruch 1, wobei, falls die Qualität der drahtlosen Verbindung zwischen der Ausgabeeinheit der Direktröntgenplatte und der Empfangseinheit der radiografischen Arbeitsstation unter einem vorgegebenen Wert liegt, eine oder mehrere der folgenden Daten zur radiografischen Arbeitsstation übertragen werden: Temperatur der Direktröntgenplatte, Batteriestatus, ein Röntgenbild niedriger Auflösung.

7. Verfahren nach Anspruch 1, wobei, falls die Qualität der drahtlosen Verbindung zwischen der Ausgabeeinheit der Direktröntgenplatte und der Empfangseinheit der radiografischen Arbeitsstation unter einem vorgegebenen Wert liegt, überprüft wird, ob das Röntgenbild im Speicher der Direktradiografie-Arbeitsstation abgespeichert worden ist, wobei bevorzugt dem Benutzer ein Feedback dieser Überprüfung zur Verfügung gestellt wird, wobei besonders bevorzugt dieses Feedback in Form eines durch die Direktröntgenplatte emittierten akustischen oder optischen Signals zur Verfügung gestellt wird oder über ein Signal, das von der Direktröntgenplatte zur radiografischen Arbeitsstation übertragen und sodann von letzterer dem Benutzer zur Verfügung gestellt wird.

8. Verfahren nach Anspruch 7, wobei der Kontrollstatus bezüglich der Speicherung des Röntgenbildes im Speicher der Direktradiografie-Arbeitsstation zu den zur radiografischen Arbeitsstation übertragenen Daten hinzugefügt wird.

9. Verfahren nach Anspruch 7, wobei das dem Benutzer zur Verfügung gestellte Feedback nur dann verschafft wird, wenn bei der Überprüfung festgestellt wird, dass das Röntgenbild nicht im Speicher der Direktröntgenplatte abgespeichert worden ist.

10. Direktröntgenplatte, das die folgenden Mittel umfasst:
- ein Mittel zum Detektieren der Qualität einer drahtlosen Verbindung zwischen einer Ausgabeeinheit der Direktröntgenplatte, welche das in der Direktröntgenplatte gespeicherte Röntgenbild auslesen und in drahtlose Kommunikationssignale umwandeln kann, und einer Empfangseinheit einer radiografischen Arbeitsstation, welche die durch die Ausgabeeinheit der Direktröntgenplatte übertragenen drahtlosen Kommunikationssignale empfangen kann,
- ein Mittel zum Vergleichen der Qualität der drahtlosen Verbindung zwischen der Ausgabeeinheit der Direktröntgenplatte und der Empfangseinheit der radiografischen Arbeitsstation mit einem vorgegebenen Wert,
- ein Mittel, mit dem, falls beim Vergleich festgestellt wird, dass die Qualität der drahtlosen Verbindung größer oder gleich dem vorgegebenen Wert ist, das in der Direktröntgenplatte abgespeicherte Röntgenbild in drahtlose Kommunikationssignale umgewandelt wird und diese Signale mittels der Ausgabeeinheit der Direktröntgenplatte zur Empfangseinheit der radiografischen Arbeitsstation übertragen werden,
- ein Mittel, mit dem, falls beim Vergleich festgestellt wird, dass die Qualität der drahtlosen Verbindung unter dem vorgegebenen Wert liegt, das Röntgenbild im Speicher der Direktröntgenplatte abgespeichert wird,
- wobei das Mittel zum Detektieren der Qualität der drahtlosen Verbindung so angeordnet wird, dass es die Qualität mittels eines Parameters, dessen Wert vom Signal-Rausch-Verhältnis der drahtlosen Übertragung abhängig ist, auswertet.

## Revendications

1. Procédé pour la communication sans fil entre un panneau radiographique direct et une station de travail radiographique, comprenant les étapes consistant à:
- détecter, à l'aide d'un processeur du panneau radiographique direct, la qualité d'une connexion sans fil entre une unité de sortie du panneau radiographique direct, laquelle est capable de lire l'image radiographique stockée dans le panneau radiographique direct et de la convertir en signaux de communication sans fil, et une unité de réception de la station de travail radiographique laquelle est capable de recevoir les signaux de communication sans fil émis par l'unité de sortie du panneau radiographique direct, ladite qualité étant déterminée à l'aide d'un paramètre dont la valeur dépend du rapport signal sur bruit de la connexion sans fil,
- si la qualité de la connexion sans fil entre l'unité de sortie du panneau radiographique direct et l'unité de réception de la station de travail radiographique est égale ou supérieure à une valeur prédéterminée, convertir l'image radiographique stockée dans le panneau radiographique direct en signaux de communication sans fil et transmettre ces signaux à l'unité de réception de la station de travail radiographique à l'aide de l'unité de sortie du panneau radiographique direct,
- si la qualité de la connexion sans fil entre l'unité de sortie du panneau radiographique direct et l'unité de réception de la station de travail radiographique est inférieure à une valeur prédéterminée, stocker l'image radiographique dans la mémoire du panneau radiographique direct.

2. Procédé selon la revendication 1, **caractérisé en ce que** le paramètre est le rapport signal sur bruit de la transmission sans fil.

3. Procédé selon la revendication 1, **caractérisé en ce que**, si la qualité de la connexion sans fil entre l'unité de sortie du panneau radiographique direct et l'unité de réception de la station de travail radiographique est inférieure à une valeur prédéterminée, les images radiographiques stockées dans la mémoire du panneau radiographique direct sont converties en signaux de communication sans fil et transmises vers l'unité de réception de la station de travail radiographique à l'aide de l'unité de sortie du panneau radiographique direct dès que la qualité sera de nouveau égale ou supérieure à la valeur prédéterminée.

4. Procédé selon la revendication 1, **caractérisé en ce que**, si le panneau radiographique direct est équipé d'une unité de sortie comprenant de multiples antennes pour communication sans fil, la qualité de la connexion sans fil entre chacune de ces antennes et la station de travail radiographique est détectée et la valeur de qualité la plus haute détectée est utilisée pour être comparée à la valeur prédéterminée de la qualité de la connexion sans fil entre le panneau radiographique direct et la station de travail radiographique.

5. Procédé selon la revendication 1, **caractérisé en ce que**, si le panneau radiographique direct est équipé d'une unité de sortie comprenant de multiples antennes, le procédé tel que décrit dans les deux premières étapes de la revendication 1 est répété et l'image radiographique ne sera stockée dans la mémoire du panneau radiographique direct que lorsque chaque valeur de qualité ainsi détectée de la connexion sans fil entre chacune de ces antennes et la station de travail radiographique est inférieure à la valeur prédéterminée.

6. Procédé selon la revendication 1, **caractérisé en ce que**, si la qualité de la connexion sans fil entre l'unité de sortie du panneau radiographique direct et l'unité de réception de la station de travail radiographique est inférieure à une valeur prédéterminée, une ou plusieurs des données citées ci-après est transmise à la station de travail radiographique: température du panneau radiographique direct, état de la batterie, une image radiographique à basse résolution.

7. Procédé selon la revendication 1, **caractérisé en ce que**, si la qualité de la connexion sans fil entre l'unité de sortie du panneau radiographique direct et l'unité de réception de la station de travail radiographique est inférieure à une valeur prédéterminée, il est vérifié si l'image radiographique a été stockée dans la mémoire de la station de travail de radiographie directe, après quoi, de préférence, un feedback de cette vérification est fourni à l'utilisateur, ledit feedback étant fourni, de manière plus préférée, sous forme d'un signal acoustique ou optique émis par le panneau radiographique direct ou par le biais d'un signal transmis à partir du panneau radiographique direct à la station de travail radiographique et retransmis ensuite à l'utilisateur par cette dernière.

8. Procédé selon la revendication 7, **caractérisé en ce que** l'état de vérification relatif au stockage de l'image radiographique dans la mémoire de la station de travail de radiographie directe est ajouté aux données transmises à la station de travail radiographique.

9. Procédé selon la revendication 7, **caractérisé en ce que** ledit feedback fourni à l'utilisateur n'est fourni que si la vérification révèle que l'image radiographique n'a pas été stockée dans la mémoire du panneau radiographique direct.

10. Panneau radiographique direct comprenant les moyens suivants:
- un moyen servant à détecter la qualité d'une connexion sans fil entre une unité de sortie du panneau radiographique direct laquelle est capable de lire l'image radiographique stockée dans le panneau radiographique direct et de la convertir en signaux de communication sans fil, et une unité de réception d'une station de travail radiographique laquelle est capable de recevoir les signaux de communication sans fil transmis par l'unité de sortie du panneau radiographique direct,
- un moyen servant à comparer la qualité de la connexion sans fil entre l'unité de sortie du panneau radiographique direct et l'unité de réception de la station de travail radiographique à une valeur prédéterminée,
- un moyen servant à convertir, si la vérification révèle que la qualité de la connexion sans fil est égale ou supérieure à la valeur prédéterminée, l'image radiographique stockée dans le panneau radiographique direct en signaux de communication sans fil et transmettre ces signaux, à l'aide de l'unité de sortie du panneau radiographique direct, à l'unité de réception de la station de travail radiographique,
- un moyen servant à stocker, si la vérification révèle que la qualité de la connexion sans fil est inférieure à la valeur prédéterminée, l'image radiographique dans la mémoire du panneau radiographique direct,
- ledit moyen de détection de la qualité de la connexion sans fil étant disposé de façon à évaluer la qualité à l'aide d'un paramètre dont la valeur dépend du rapport signal sur bruit de la transmission sans fil.
